(19) Europäisches Patentamt
European Patent Office
Office européen des brevets

(11) EP 3 171 154 A1

(12) EUROPÄISCHE PATENTANMELDUNG

(43) Veröffentlichungstag:
24.05.2017 Patentblatt 2017/21

(51) Int Cl.:
G01N 21/00 (2006.01)          G01N 33/96 (2006.01)
G01N 33/70 (2006.01)

(21) Anmeldenummer: 16198632.8

(22) Anmeldetag: 14.11.2016

(84) Benannte Vertragsstaaten:
AL AT BE BG CH CY CZ DE DK EE ES FI FR GB
GR HR HU IE IS IT LI LT LU LV MC MK MT NL NO
PL PT RO RS SE SI SK SM TR
Benannte Erstreckungsstaaten:
BA ME
Benannte Validierungsstaaten:
MA MD

(30) Priorität: 23.11.2015 DE 102015120216

(71) Anmelder: B. Braun Avitum AG
34212 Melsungen (DE)

(72) Erfinder:
• ALIC, Miran
  8001 Zürich (CH)
• Dr. STROHHÖFER, Christof
  34123 Kassel (DE)
• JANIK, Waldemar
  34212 Melsungen (DE)

(74) Vertreter: Winter, Brandl, Fürniss, Hübner,
Röss, Kaiser, Polte - Partnerschaft mbB
Patent- und Rechtsanwaltskanzlei
Alois-Steinecker-Strasse 22
85354 Freising (DE)

(54) **VERFAHREN ZUR KALIBRIERUNG EINES MESSSIGNALS UND ZUM NACHFÜHREN EINER QUANTITATIVEN GRÖSSE**

(57) Ein Verfahren zur Kalibrierung eines Messsignals und/oder zur Nachführung einer quantitativen Größe beinhaltet das Vermessen eines mit einer Konzentration in einer Lösung vorliegenden Analyten, der eine vorbestimmte Abklingkinetik aufweist und das Erzeugen eines kontinuierlichen Messsignals mit einer zu der des Analyten zumindest entsprechenden Abklingkinetik. Die Abklingkinetik des Messsignals und die Abklingkinetik des Analyten werden unter Verwendung zumindest eines vorbestimmten Kalibrierpunkts beider Abklingkurven korreliert. Nachfolgende Konzentrationswerte des Analyten werden sodann aus dem Messsignal berechnet.

FIG. 6

**Beschreibung**

Die Erfindung betrifft ein Verfahren zur Kalibrierung eines Messsignals und zum Nachführen einer quantitativen Größe.

Bei der quantitativen Messung chemischer Komponenten, beispielsweise von Analyten wie etwa der urämischen Toxine Kreatinin und Harnsäure, werden regelmäßig Additive eingesetzt, die dazu dienen, die Eindeutigkeit von Stoffen, wie beispielsweise Enzyme, Farbstoffe oder Nanopartikel, oder von Stoffen, die allgemeine Umgebungseigenschaften der Messlösung, wie beispielsweise deren pH-Wert, verändern, zu gewährleisten. Solche Additive können auf Analyten spezialisiert sein (d.h. diese "targeten"). Der Einsatz von Additiven hat zur Folge, dass eine entsprechende Vorrichtung eine bestimmte Menge an Additiven vorhalten muss. Dies ist mit Kosten verbunden.

Die Berechnung von Konzentrationen ausgewählter Stoffe erfolgt in der Regel durch die Verwertung einer Messgröße, beispielsweise einer Leitfähigkeit (für etwa Elektrolyte) oder einer Extinktion (für etwa Kreatinin und/oder Harnsäure). Hierbei ist das Messsignal häufig eine Überlagerung von Zuträgen verschiedener Stoffe. In diesem Fall besteht die Aufgabe des Messsystems darin, die zu messende Größe aus einem entsprechenden Messsignal zu extrahieren und damit von den interferierenden Stoffen zu trennen. In der Praxis erfolgt dies durch beispielsweise den Einsatz einer Kombination chemischer Additive (Enzyme) und Farbstoffe. Die Veränderung der Enzym-Farbstoff-Zusammenwirkung ist dann proportional zu der Konzentration des gesuchten Stoffes, wodurch eine einfache Umrechnung möglich wird. Nachteilig ist allerdings für jede Messung einer Konzentration der erforderliche Einsatz von Enzymen und Farbstoffen.

Insbesondere in der Dialyse, bei der es über den Dialysator zu einem Stofftransfer zwischen dem Blut und der Dialysierflüssigkeit kommt und die vereinfacht als ein Prozess beschreibbar ist, in dem Substanzen (urämische Toxine, Elektrolyte) aus dem Blut über eine Membran (Dialysator) in eine aufbereitete Flüssigkeit (Dialysierflüssigkeit) gebracht werden, ist ein kontinuierlicher Einsatz von Additiven problematisch. Die in der Dialyse erhaltene verbrauchte Dialysierflüssigkeit (Dialysat) als eine Pufferlösung, in welcher häufig Messungen von Messsignalen vorgenommen werden, ist ein chemisches Abbild des Blutes und mit einer hohen Anzahl von urämischen Toxinen belastet. Üblicherweise werden dort optische Sensoren eingesetzt, um eine Entfernungsrate (z.B. Kt/V) zu berechnen. Falls ein entsprechendes Additiv vorhanden ist, können damit aber auch qualitative Größenwerte, wie beispielsweise die Konzentration eines Analyten, gewonnen werden. Allerdings ist in diesem Umfeld der Einsatz von Additiven sehr umständlich und ist deren Lagerung derart aufwendig, dass bislang kein quantitativ messender Sensor in der Dialyse etabliert werden konnte.

Der Erfindung liegt daher als eine Aufgabe zugrunde, die vorgenannten Nachteile zu beseitigen und ein Verfahren zu schaffen, mit welchem bei einer (quasi-) kontinuierlichen quantitativen Messung von Substanzen eine Einsparung von Additiven erzielbar ist.

Diese Aufgabe wird erfindungsgemäß gelöst durch ein Verfahren mit den Merkmalen des Anspruchs 1. Vorteilhafte Weiterbildungen der Erfindung sind Gegenstand der beigefügten Unteransprüche.

Der Erfindung liegt die allgemeine Idee zugrunde, durch eine geeignete Abbildung eines Konzentrationswertes auf einen Signalwert am Anfang einer Therapie (Kalibrierung) die nachfolgenden Konzentrationswerte durch Nachführen eines Rohsignals berechenbar zu machen. Außer der Zugabe eines Additivs zu Beginn einer Therapie (d.h. zumindest einer einmaligen Zugabe) ist sodann keine weitere Erfassung eines Signals in einer (beispielsweise bezüglich des pH-Werts) geänderten Umgebung erforderlich. Es können jedoch durchaus weitere Messungen durchgeführt werden. In diesem Fall können weitere Größen, wie beispielsweise das Verteilungsvolumen oder Transferbehinderungsmechanismen, bestimmt werden.

Entlang der vorgenannten allgemeinen Idee kann so bei geeigneter Kalibrierung unter Nutzung von Eigenschaften dahingehend, dass eine Entfernung kleinmolekularer Substanzen aus beispielsweise Blut in beispielsweise verbrauchte Dialysierflüssigkeit einer festgelegten Kinetik folgt, eine einmalige Messung einer Konzentration zu Beginn einer Therapie ausreichen, um nachfolgende Konzentrationswerte ohne Einsatz von Additiven zu bestimmen. Die vorgenannten Nachteile können somit durch eine geschickte Kalibrierung umgangen werden.

Die Erfindung ist insbesondere dahingehend vorteilhaft, dass ein geringerer Einsatz von Additiven erforderlich ist, eine Kosten- und Materialersparnis erzielt wird, geringerer Verschleiß auftritt und erhöhte Sicherheit erhalten wird.

Im Einzelnen werden die vorstehenden Vorteile realisiert und die Aufgabe gelöst durch ein Verfahren zur Kalibrierung eines Messsignals und/oder zur Nachführung einer quantitativen Größe, beinhaltend: Vermessen eines mit einer Konzentration in einer Lösung vorliegenden Analyten, der eine vorbestimmte Abklingkinetik aufweist und Erzeugen eines kontinuierlichen Messsignals mit einer zu der des Analyten zumindest entsprechenden Abklingkinetik; Korrelieren der Abklingkinetik des Messsignals und der Abklingkinetik des Analyten unter Verwendung zumindest eines vorbestimmten Kalibrierpunkts beider Abklingkurven; und Berechnen nachfolgender Konzentrationswerte des Analyten aus dem Messsignal.

In anderen Worten werden die vorstehenden Vorteile realisiert und die Aufgabe gelöst durch ein Verfahren zur Kalibrierung eines Messsignals und/oder zur Nachführung einer quantitativen Größe, beinhaltend: Erzeugen zumindest eines Messwerts eines in einer Lösung vorliegenden Analyten, der eine vorbestimmte, einen abklingenden Konzentrationsverlauf des Analyten definierende Abklingkurve aufweist, als eine quantitative Größe; Erzeugen eines zumindest quasi-kontinuierlichen Messsignals mit einem zu dem abklingenden Konzentrationsverlauf des Analyten zumindest entsprechenden abklingenden Verlauf basierend auf einem Sensorsignal, wobei der Analyt einen Teil des Messsignals bestimmt und ein verbleibender Teil des Messsignals nicht von dem Analyten bestimmt wird; Korrelieren des Verlaufs des Messsignals und der Abklingkurve des Analyten unter Verwendung zumindest eines vorbestimmten Kalibrierpunkts auf jeweils der Abklingkurve des Analyten und dem Verlauf des Messsignals; und Nachführen der quantitativen Größe durch Berechnen nachfolgender Konzentrationswerte des Analyten aus dem Messsignal.

Bevorzugt werden weiters folgende Verfahrensschritte durchgeführt: Auswählen eines für eine erste quantitative Größe erhaltenen ersten Messsignalverlaufs aus einer Vielzahl von für die erste quantitative Größe und für zumindest eine zweite quantitative Größe erhaltenen Messsignalverläufen; Durchführen einer Kalibrierung für zumindest einen Messwert eines zweiten Messsignalverlaufs bei der zweiten quantitativen Größe aus der Messung des entsprechenden Messwerts bei der ersten quantitativen Größe; Prüfen aller der bei der ersten quantitativen Größe und der zweiten quantitativen Größe erhaltenen Messwerte auf linearen Zusammenhang oder zumindest hinreichend, das heißt näherungsweise vorliegenden, linearen Zusammenhang; und dann, wenn ein linearer oder zumindest hinreichend linearer Zusammenhang besteht, Berechnen zumindest eines nachfolgenden Messwerts für die zweite quantitative Größe aus dem ersten Messsignalverlauf für die erste quantitative Größe.

Bevorzugt ist die erste quantitative Größe ein erster pH-Wert, ist die zweite quantitative Größe ein zweiter pH-Wert und sind die Messwerte Extinktionswerte.

Bevorzugt liegt dem ersten und dem zweiten Messsignalverlauf eine gleiche Wellenlänge von zur Ermittlung der Extinktionswerte verwendeten Analyselichts zugrunde.

Bevorzugt werden zur Berechnung eines Verteilungsvolumens weiters folgende Verfahrensschritte durchgeführt: Messen der lösungsseitigen Konzentration eines in der Lösung vorhandenen gewünschten Analyten; Korrelieren eines Extinktionssignals und/oder zumindest eines Extinktionswerts mit der gemessenen Konzentration; Messen des Extinktionssignals und/oder zumindest eines Extinktionswerts als Funktion einer Zeit, die während einer Therapie verstreicht und Erhalten eines kontinuierlich vorliegenden Messsignals; Berechnen eines Konzentrationssignals und/oder zumindest eines Konzentrationswerts zu vorbestimmten, diskreten Zeitpunkten aus dem kontinuierlich vorliegenden Messsignal; Messen der blutseitigen Konzentration des Analyten; Bestimmen einer entfernten Gesamtmasse; und Ermitteln des Verteilungsvolumens aus der bestimmten Gesamtmasse, der lösungsseitigen Konzentration des Analyten und der blutseitigen Konzentration des Analyten.

Bevorzugt wird das Verteilungsvolumen in Übereinstimmung mit der Beziehung $V = m / (C1 - C2)$ errechnet, worin C1 eine Konzentration des Analyten zu Beginn einer Therapiedauer und C2 eine Konzentration des Analyten am Ende der Therapiedauer sind und m die zwischen den beiden gemessenen Konzentrationen entfernte Gesamtmasse des Analyten und/oder dessen Integral ist.

Bevorzugt werden zur Erfassung einer fluidbezogenen Transportbeeinträchtigung weiters folgende Verfahrensschritte durchgeführt: Messen der lösungsseitigen Konzentration eines in der Lösung vorhandenen gewünschten Analyten; Korrelieren eines Extinktionssignals und/oder zumindest eines Extinktionswerts mit der gemessenen Konzentration; Messen des Extinktionssignals und/oder zumindest eines Extinktionswerts als Funktion einer Zeit, die während einer Therapie verstreicht und Erhalten eines kontinuierlich vorliegenden Messsignals; Berechnen eines Konzentrationssignals und/oder zumindest eines Konzentrationswerts zu vorbestimmten, diskreten Zeitpunkten aus dem kontinuierlich vorliegenden Messsignal; Vergleichen des berechneten Konzentrationssignals und/oder zumindest eines Konzentrationswerts mit der gemessenen Konzentration; und Ermitteln eines Vorhandenseins einer Transportbeeinträchtigung bei Ungleichheit der miteinander verglichenen Signale und/oder Werte.

Bevorzugt ist die Lösungsseite eine Dialysierflüssigkeitsseite.

Bevorzugt wird das Verfahren unter einer Bedingung einer voreingestellten Clearance von 100% während der Therapiedauer durchgeführt.

Bevorzugt wird sowohl die lösungsseitige Konzentration des Analyten als auch die blutseitige Konzentration des Analyten bei einer voreingestellten Clearance von 100% gemessen.

Bevorzugt wird/werden ein quantitatives Verfahren und/oder ein pH-Shift-Verfahren durchgeführt.

Bevorzugt wird/werden gleichzeitig zu dem quantitativen Verfahren ein Extinktionssignal und/oder zumindest ein Extinktionswert gemessen.

Bevorzugt wird/werden ein während der Durchführung des quantitativen Verfahrens bereits ermitteltes Extinktionssignal und/oder ein bereits ermittelter Extinktionswert übernommen.

Bevorzugt wird der Konzentrationswert über einen Umrechnungsfaktor und/oder eine Formelbeziehung mit dem Extinktionssignal und/oder dem zumindest einen Extinktionswert korreliert.

Bevorzugt wird die entfernte Gesamtmasse durch Bildung eines Integrals aus den berechneten Werten bestimmt.

Bevorzugt werden zur Bestimmung eines in einem Körper enthaltenen überschüssigen Wassers weiters die Schritte durchgeführt: Bestimmen der Gesamtwassermenge, des Gesamtgewichts und des Fettgehalts des Körpers;

Ermitteln der Magermasse durch Subtrahieren des Fettgehalts und der Gesamtwassermenge von dem Gesamtgewicht; Aufteilen der Magermasse in einen vorbestimmten Festbestandteil und einen vorbestimmten Flüssigkeitsanteil, wobei der Flüssigkeitsanteil als eine optimale Flüssigkeitsmenge angenommen wird; Gleichsetzen des ermittelten Verteilungsvolumens und der Gesamtwassermenge des Körpers; und Ermitteln der Menge überschüssigen Wassers durch Subtrahieren der optimalen Flüssigkeitsmenge von dem ermittelten Verteilungsvolumen.

Alternativ bevorzugt werden zur Bestimmung eines in einem Körper enthaltenen überschüssigen Wassers weiters die Schritte durchgeführt: Bestimmen der Gesamtwassermenge und des Gesamtgewichts des Körpers; Ermitteln der Magermasse durch direkte Messung unter Verwendung eines vorbestimmten Kreatininkinetikmodells; Aufteilen der Magermasse in einen vorbestimmten Festbestandteil und einen vorbestimmten Flüssigkeitsanteil, wobei der Flüssigkeitsanteil als eine optimale Flüssigkeitsmenge angenommen wird; Gleichsetzen des ermittelten Verteilungsvolumens und der Gesamtwassermenge des Körpers; und Ermitteln der Menge überschüssigen Wassers durch Subtrahieren der optimalen Flüssigkeitsmenge von dem ermittelten Verteilungsvolumen.

Die Erfindung wird nachstehend anhand bevorzugter Ausführungsbeispiele unter Bezugnahme auf die beigefügte Zeichnung näher beschrieben. Es zeigen:

Fig. 1 mit einer Kurve (a) eine schematische Darstellung eines Verlaufs der Konzentration eines Analyten in verbrauchter Dialysierflüssigkeit als Funktion der Zeit, mit einer Kurve (b) eine schematische Darstellung eines Verlaufs eines Signals wie beispielsweise einer Absorbanz eines Sensors als Funktion der Zeit und mit einer Kurve (c) ein Beispiel einer Kalibrierung, bei dem ein Konzentrationswert am Anfang einer Therapie genutzt wird, um aus dem Messsignal Konzentrationen des Analyten zu berechnen;

Fig. 2 beispielhafte UV-VIS Absorptionsspektren verbrauchter Dialysierflüssigkeit für jeweils einen pH-Wert von 7,3 und einen pH-Wert von 3,8 an vier äquidistanten Zeitpunkten einer 230 Minuten dauernden Hämodialysetherapie;

Fig. 3 einen Scatterplot von Extinktionsmessungen bei einem pH-Wert von 7,3 und einem pH-Wert von 3,8 mit Kennzeichnung des Ergebnisses der Regression für alle Punkte des Plots und Darstellung einer Ausgleichgeraden für einen Punkt (einschließlich Nulldurchgang);

Fig. 4 einen Scatterplot zwischen Extinktionswerten bei 254 nm und 290 nm und bei einem pH Wert von 7,3;

Fig. 5 eine schematisch skizzierte Darstellung für eine externe Kalibrierung;

Fig. 6 eine schematische Darstellung eines typischen Signalverlaufs einer optischen Extinktion während einer Dialyse mit einem Kalibrierpunkt zu Beginn der Therapie, anschließend berechneten Punkten von Konzentrationen im Messsignal sowie das Integral unter der Funktion bzw. Punkten bzw. dem Messsignalverlauf;

Fig. 7 eine Darstellung zur Erläuterung eines Nachverfolgungs- bzw. Tracking-Verfahrens für die Bestimmung eines Verteilungsvolumens;

Fig. 8 eine Darstellung eines Signalverlaufs einer optischen Extinktionsmessung in Dialysierflüssigkeit sowie eine tatsächliche Entwicklung der blutseitigen Konzentration des Analyten im Falle eines Transportproblems an der Dialysatormembran;

Fig. 9 eine schematische Darstellung in Bezug auf die Bestimmung von in einem Dialysepatienten vorhandenen überschüssigen Wassers sowie des Gesamtkörperwassers (Fluidmanagement); und

Fig. 10 eine Umformung der Darstellung in Fig. 9.

In der nachfolgenden Figurenbeschreibung werden in den einzelnen Figuren gleiche oder gleichwirkende Schritte, Elemente und/oder Komponenten gleich und/oder mit denselben Bezugszeichen bezeichnet und zweckmäßig nicht redundant beschrieben. In Fällen, in welchen ein nachfolgendes Ausführungsbeispiel funktionell zumindest einem vorangehenden entspricht, d.h. entsprechende Funktionen, Anordnungen und/oder Verfahrens- oder Betriebsabläufe gleichermaßen umfasst sind, wird zweckmäßig nur auf Unterschiede eingegangen.

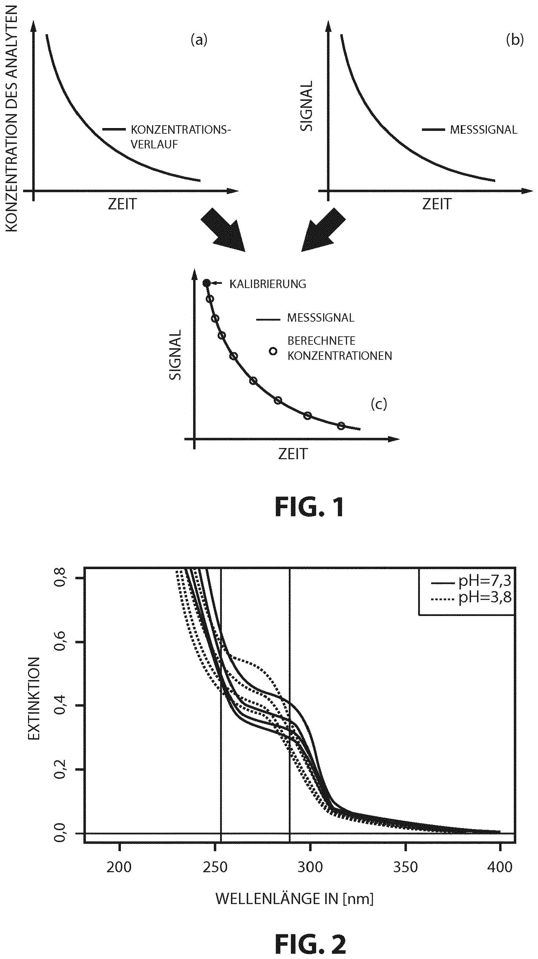

Fig. 1 zeigt unter (a) eine schematische Darstellung eines Verlaufs der Konzentration eines Analyten in verbrauchter Dialysierflüssigkeit als Funktion der Zeit, unter (b) eine schematische Darstellung eines Verlaufs eines Signals wie beispielsweise einer Absorbanz eines Sensors als Funktion der Zeit und unter (c) ein Beispiel einer Kalibrierung, bei dem ein Konzentrationswert am Anfang einer Therapie genutzt wird, um aus dem Messsignal Konzentrationen des Analyten zu berechnen.

Fig. 1 zeigt somit vereinfacht drei verschiedene Kurven. Kurve (a) stellt den Verlauf der Konzentration eines Analyten und Kurve (b) den Verlauf eines Messsignals dar. Das Messsignal und der Analyt sind derart verknüpft, dass der Analyt einen Teil des Messsignals bestimmt. Den verbleibenden Anteil des Messsignals bestimmen andere, interferierende Substanzen, so dass eine direkte Umrechnung des Messsignals in die richtige Konzentration eines bestimmten Analyten zunächst nicht möglich ist. In der Praxis werden daher Additive eingesetzt, um eine eindeutige Zuweisung zu ermöglichen. Auch wenn die Konzentration eines Analyten aus dem alleinig vorhandenen Messsignal nicht berechenbar ist, bestehen Situationen, in welchen das Messsignal und die Konzentration des Analyten über eine gleiche oder eine ähnliche Abklingkinetik verfügen und so miteinander in Verbindung gebracht werden können. In diesem Fall können weitere Konzentrationen aus dem quasi-kontinuierlich vorhandenen Messsignal jederzeit berechnet werden.

In Fig. 1 ist mit der Kurve (c) ein solcher Fall dargestellt, bei dem eine Kalibrierung ausreichend ist, um das Messsignal in eine Konzentration umzurechnen. Durch eine anfängliche Kalibrierung zu Beginn einer Therapie können auf diese Weise aufwendige Messungen der Konzentration vermieden werden. Dabei kann ein Kalibrierpunkt durchaus durch eines der quantitativen, auf Additiven basierenden Verfahren oder durch andere etablierte Verfahren bestimmt sein, so lange anschließende bzw. nachfolgende Konzentrationswerte sodann aus dem Messsignal berechnet werden können.

Fig. 2 zeigt beispielhafte UV-VIS Absorptionsspektren (Absorptionsspektren einer Spektroskopie, die elektromagnetische Wellen des ultravioletten und des sichtbaren Lichts nutzt) verbrauchter Dialysierflüssigkeit für jeweils einen pH-Wert von 7,3 und einen pH-Wert von 3,8 an vier äquidistanten Zeitpunkten einer 230 Minuten dauernden Hämodialysetherapie.

Bezogen auf Fig. 2 zielt gemäß einem ersten Ausführungsbeispiel ein Verfahren zur Kalibrierung eines Messsignals und zum Nachführen einer quantitativen Größe auf Einsparungen von Additiven bei der Messung von Kreatinin und Harnsäure auf der Grundlage einer pH-Wert-Verschiebung ab. Mit Hilfe der Verschiebung des pH-Werts von verbrauchter Dialysierflüssigkeit durch Änderung des pH-Wertes des Lösungsmittels, zum Beispiel durch Zugabe eines sauren Mediums, kann eine Berechnung von Kreatinin- und Harnsäurekonzentrationen durchgeführt werden. Dazu werden beispielsweise zwei Extinktionswerte bei einer Wellenlänge von 254 nm erfasst, wobei zwischen der Messung des ersten und zweiten Extinktionswerts ein Additiv zugegeben wird. Das Additiv beeinflusst das zweite Extinktionssignal derart, dass die Differenz zwischen dem ersten und dem zweiten Wert bzw. Signal proportional zur Konzentration des Analyten ist. Die Zugabe des Additivs ist in diesem Verfahren für jede Messung der Konzentration nötig.

Gemäß dem vorliegenden Ausführungsbeispiel des erfindungsgemäßen Verfahrens kann die Häufigkeit der Zugabe(n) des Additivs verringert werden.

Wie in Fig. 2 beispielhaft gezeigt, sind z.B. 4 (von 16) Paaren von UV-VIS Spektren, die bei einer Dialysetherapie entnommen bzw. aufgezeichnet wurden, dargestellt. Vier der gezeigten acht Spektren beziehen sich auf einen pH-Wert von 7,3, und vier weitere der gezeigten acht Spektren beziehen sich auf einen pH-Wert von 3,8. Ersichtlich korrelieren die einzelnen Spektren hinsichtlich ihres Abklingens bzw. ihrer Abklingkinetik hochgradig miteinander.

Gemäß dem vorliegenden Ausführungsbeispiel wird durch Auswählen bzw. auf Grundlage der hier speziellen Wellenlänge von 254 nm, Durchführen einer anfänglichen Kalibrierung der Extinktionswert bei dem pH-Wert 3,8 aus der Messung des Extinktionswerts bei dem pH-Wert 7,3 und Prüfen der Extinktionswerte bei dem pH Wert von 7,3 und dem pH-Wert von 3,8 auf linearen oder zumindest hinreichend linearen Zusammenhang, und dann, wenn ein linearer oder zumindest hinreichend linearer Zusammenhang besteht, der Extinktionswert bzw. eine Mehrzahl benötigter Extinktionswerte aus der Messung für den pH-Wert 7,3 ermittelt oder berechnet.

In anderen Worten wird ein für eine erste quantitative Größe bzw. einen ersten pH-Wert erhaltener erster Messsignalverlauf aus einer Vielzahl von für die erste quantitative Größe bzw. den ersten pH-Wert und für zumindest eine zweite quantitative Größe bzw. einen zweiten pH-Wert erhaltenen Messsignalverläufen ausgewählt, wird eine Kalibrierung für zumindest einen Messwert, beispielsweise eines Extinktionswerts, des zweiten Messsignalverlaufs bei der zweiten quantitativen Größe bzw. dem zweiten pH-Wert aus der Messung des entsprechenden Messwerts bei der ersten quantitativen Größe bzw. dem ersten pH-Wert durchgeführt, werden alle der bei der ersten quantitativen Größe bzw. dem ersten pH-Wert und der zweiten quantitativen Größe bzw. dem zweiten pH-Wert erhaltenen Messwerte auf linearen Zusammenhang oder zumindest hinreichend, das heißt näherungsweise vorliegenden, linearen Zusammenhang geprüft und wird dann, wenn ein linearer oder zumindest hinreichend linearer Zusammenhang besteht, zumindest ein nachfolgender Messwert für die zweite quantitative Größe bzw. den zweiten pH-Wert aus dem ersten Messsignalverlauf für die erste quantitative Größe bzw. den ersten pH-Wert berechnet.

Die Kalibrierung kann hierbei derart umschrieben werden, dass zwei vorbestimmte und sich entsprechende Punkte oder Messwerte, beispielsweise Extinktionswerte, vorzugsweise zu Beginn einer Therapie und damit am Anfang entsprechender Messkurven, auf jeweils zugehörigen Messsignalverläufen in Übereinstimmung bzw. zur Deckung gebracht, d.h. sozusagen übereinandergelegt, werden. Sind diese beiden Punkte deckungsgleich, wird eine Übereinstimmung der einzelnen Abklingkinetiken der einzelnen Messkurven erfassbar. Im Falle einer Messung von Extinktionswerten liegt dem ersten und dem zweiten Messsignalverlauf vorzugsweise eine gleiche bzw. dieselbe Wellenlänge von zur Ermittlung der Extinktionswerte verwendeten Analyselichts zugrunde.

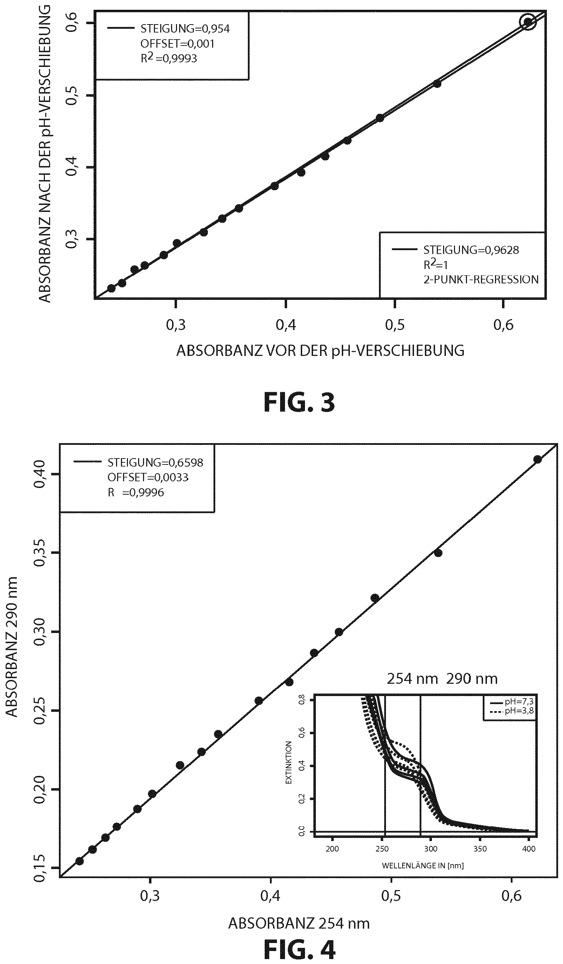

Fig. 3 zeigt zur weiteren Erklärung der Prüfung auf Linearität einen Scatterplot von Extinktionsmessungen bei einem pH-Wert von 7,3 und einem pH-Wert von 3,8 mit Kennzeichnung des Ergebnisses der Regression für alle Punkte des Plots und Darstellung einer Ausgleichgeraden für einen Punkt (einschließlich Nulldurchgang).

Im Einzelnen ist in Fig. 3 ein Scatterplot zwischen den Extinktionswerten bei pH 7,3 und pH 3,8 und der Wellenlänge 254 nm für verschiedene Zeitpunkte in der Dialyse aufgetragen. Zusätzlich sind zwei Regressionsanalysen abgebildet. Eine erste Ausgleichgerade wurde auf allen Datenpunkten berechnet, eine zweite unter Zuhilfenahme der eingekreist dargestellten Punkte und des Nulldurchgangs.

Wie in Fig. 3 ersichtlich, zeigen beide Ausgleichsgeraden ein hohes Maß an Übereinstimmung. Durch eine 2-Punkt-Kalibrierung ist es daher unabhängig davon, ob der Kalibrierung alle Punkte oder nur ein Punkt zugrunde liegt, möglich, jeden Extinktionswert bei pH 7,3 einem entsprechenden pH-Wert bei 3,8 gegenüberzustellen. Die Steigung der Ausgleichsgeraden ist in diesem Fall dann ein patientenspezifischer Kalibrierungsfaktor. Daraus folgt, dass für die Konzentrationsbestimmung ein wesentlich geringerer Einsatz von Additiven erforderlich ist und dieser darüber hinaus lediglich zu Beginn einer Therapie zu erfolgen braucht. Die Anzahl der Messpunkte, welche vorzugsweise für die Kalibrierung genutzt werden sollte, hängt von einer jeweiligen Anwendung ab und kann je nach Ausführung einen oder mehrere Datenpunkte enthalten.

In einem zweiten Ausführungsbeispiel des erfindungsgemäßen Verfahrens wird ein weiterer starker Zusammenhang bzw. eine weitere starke Korrelation zwischen Extinktionswerten genutzt. Hierbei wird auf der Grundlage von Co-Linearitäten zwischen einzelnen Wellenlängen eine freie Wahl der Analysewellenlänge des Messsystems gewährleistet. Hierdurch wird der Einsatz verschiedener Lichtquellen möglich, wodurch ein erheblicher Kostenvorteil erzielt werden kann.

Fig. 4 zeigt hierzu einen beispielhaften Scatterplot zwischen Extinktionswerten bei 254 nm und 290 nm und bei einem pH Wert von 7,3, in welchem erneut eine sehr starke Korrelation erkennbar ist. Aufgrund dessen ist es möglich, ein Signal oder eine Kalibrierung das bzw. die ursprünglich bei der (Analyselicht-) Wellenlänge 254 nm gemessen bzw. durchgeführt wurde, auf die Wellenlänge 290 nm zu übertragen. Dadurch wird es möglich, für die Nachverfolgung bzw. Nachführung oder das Tracking Lichtquellen einzusetzen, die eine wesentlich höhere Lebensdauer haben.

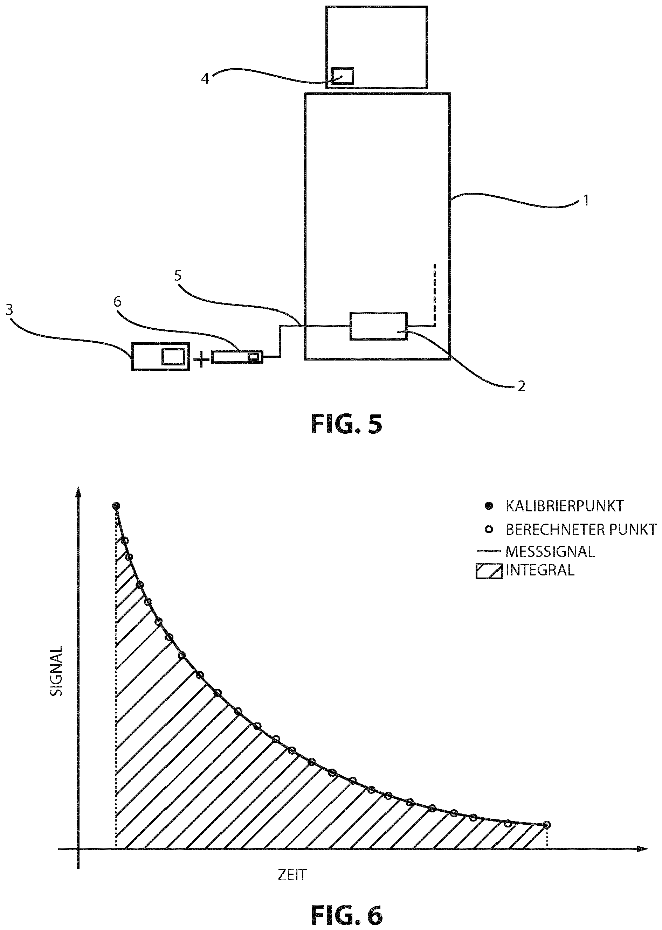

Fig. 5 zeigt eine schematisch skizzierte Darstellung für eine externe Kalibrierung eines trackingfähigen Messsystems gemäß einem dritten Ausführungsbeispiel.

Vorstehend wurden Verfahren zur Konzentrationsbestimmung ausgewählter Substanzen durch ein einmaliges Kalibrieren und anschließendes "Tracking" eines Messsignals beschrieben, wobei die erforderlichen Kalibrierungen durch interne Komponenten einer Maschine ausgeführt werden. Prinzipiell kann eine Kalibrierung aber auch mittels einer externen Einrichtung erfolgen.

Gemäß Fig. 5 kann beispielsweise eine als externer Sensor konfigurierte externe Vorrichtung 3, hier beispielsweise in Kombination mit einem Teststreifen 6, aus dem Ausfluss 5 einer Dialysemaschine 1 austretende verbrauchte Dialysierflüssigkeit vermessen. Die Konzentration eines ausgewählten Analyten kann von der externen Vorrichtung 3 abgelesen und in die Dialysemaschine eingetragen werden. Dies kann entweder durch manuelle Eingabe oder mittels einer automatisierten Übertragung durch eine drahtlose und/oder drahtgebundene Schnittstelle (beispielsweise LAN, WLAN) geschehen. Der Abgleich zwischen einem relativen Messsignal und einer quantitativ erfassten Größe kann sodann maschinenintern in Übereinstimmung mit beispielsweise einer zuvor hinterlegten Regel durchgeführt werden. Die Dialysemaschine 1 beinhaltet ferner einen (internen) Sensor 2 und eine Anzeigeeinrichtung 4 zur Anzeige von Daten und/oder Werten. Dadurch wird es der Dialysemaschine 1 möglich, weitere Konzentrationswerte des Analyten ohne zusätzlichen Einsatz von anderen Techniken und/oder Vorrichtungen auszugeben.

Fig. 6 zeigt eine schematische Darstellung eines beispielhaft typischen Signalverlaufs einer optischen Extinktion (Signal, beispielsweise Absorbanz, entlang der Ordinate über der Zeit entlang der Abszisse) während einer Hämodialysetherapie, mit einem Kalibrierpunkt zu Beginn der Therapie, anschließend berechneten Punkten von Konzentrationen im Messsignal sowie das Integral unter der Funktion bzw. Punkten bzw. dem Messsignalverlauf, gemäß einem vierten Ausführungsbeispiel.

Mittels des vorstehend beschriebenen Trackingverfahrens können hierbei aus dem Messsignal weitere Konzentrationen berechnet werden, auf deren Grundlage wiederum ein Integral berechenbar ist, welches letztendlich in der während der Dialyse entfernten Gesamtmenge des ausgewählten Analyten resultiert. Diese entfernte Gesamtmenge eines Analyten kann unter Verwendung weiterer Techniken zur Messung weiterer Größen genutzt werden.

Beispielsweise kann das vorstehend beschriebene Trackingverfahren zur quantitativen Messung einer blutseitigen Konzentration während einer Dialyse herangezogen werden. Durch zum Beispiel Verringerung des Blutflusses ist es möglich, der Konzentration eines ausgewählten Stoffes in verbrauchter Dialysierflüssigkeit eine Konzentration desselben im Blut zuzuordnen, wobei in diesem Fall eine Clearance von 100% erreicht werden kann. In anderen Worten weist der ausgewählte Stoff bzw. die ausgewählte Substanz in Blut und verbrauchter Dialysierflüssigkeit dieselbe Konzentration auf und entspricht somit dessen Konzentration am Blutausgang (BA) derjenigen am Dialysierflüssigkeitseingang (DE).

Mit Herleitung aus der Massenbilanz (vereinfacht unter Weglassung der Ultrafiltrationsrate (UFR):

$$Q_b \bullet BE + Q_d \bullet DE = Q_b \bullet BA + Q_d \bullet DA \qquad \text{(Gleichung 1)}$$

worin $Q_b$ der Blutfluss ist, $Q_d$ der Dialysierflüssigkeitsfluss ist, BE die Bluteingangskonzentration ist, DE die Dialysierflüssigkeitseingangskonzentration ist, BA die Blutausgangskonzentration ist, und DA die

Dialysierflüssigkeitsausgangskonzentration ist, ergibt sich bei Umstellung nach der Bluteingangskonzentration BE:

$$BE = BA + Q_d/Q_b \bullet (DA - DE) \qquad \text{(Gleichung 2)}$$

Im Falle einer Clearance von 100%, d.h. bei geringem Blutfluss, nähert sich die Blutausgangskonzentration der Dialysierflüssigkeitseingangskonzentration an:

$$BE = DE + Q_d/Q_b \bullet (DA - DE) \qquad \text{(Gleichung 3)}$$

Falls ein gesuchter Stoff in frischer Dialysierflüssigkeit nicht vorhanden ist, d.h. dessen Konzentration DE = 0 ist, ergibt sich:

$$BE = Q_d/Q_b \bullet DA \qquad \text{(Gleichung 4)}$$

Auf der Grundlage weiterführender Annahmen sind daraus weitere Größen bestimmbar, wie beispielsweise das blutseitige Verteilungsvolumen, dem in der Dialyse bei der Bestimmung der Dialysedosis eine wichtige Funktion bzw. Rolle zukommt. Insoweit kann das vorstehend beschriebene Trackingverfahren auch für die Bestimmung des blutseitigen Verteilungsvolumens herangezogen werden.

Beispielsweise ist es möglich, durch Kombination der Detektion der blutseitigen Konzentration eines ausgewählten Stoffes mit dem vorstehend beschriebenen Trackingverfahren und der Bestimmung der entfernten Gesamtmasse des ausgewählten Stoffes das Verteilungsvolumen V zu errechnen:

$$C_1 \bullet V - C_2 \bullet V = m \qquad \text{Gleichung (5)}$$

$$V = m / (C1 - C2) \qquad \text{Gleichung (6)}$$

worin C1 und C2 zwei Blutkonzentrationen vorzugsweise zu Beginn und am Ende einer Therapie sind und m die zwischen den beiden gemessenen Konzentrationen entzogene bzw. entfernte Gesamtmasse des Stoffs bzw. der Substanz, d.h. dessen bzw. deren Integral, ist.

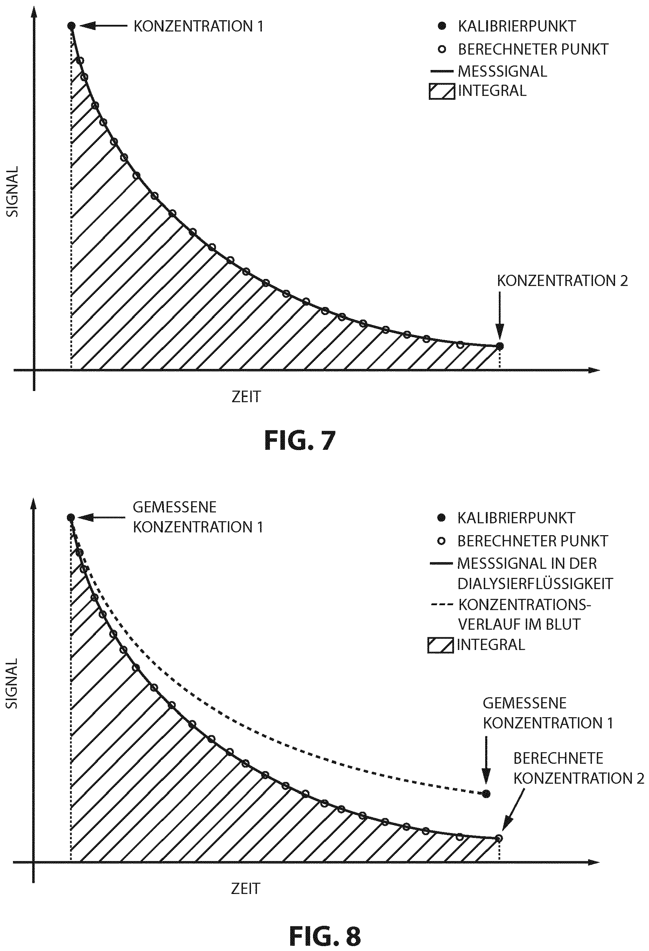

Fig. 7 zeigt im vorgenannten Zusammenhang eine Darstellung zur Erläuterung eines Nachverfolgungs- bzw. Tracking-Verfahrens für die Bestimmung eines Verteilungsvolumens und im Einzelnen die wesentlichen, zur Berechnung des Verteilungsvolumens V erforderlichen Messgrößen.

Unter der Voraussetzung, dass während einer Therapiebehandlung durch geeignetes Festlegen des Blutflusses (beispielsweise auf 50 ml/min) und des Dialysierflüssigkeitsflusses (beispielsweise auf 500 ml/min) eine Clearance von 100% zwischen der Dialysierflüssigkeitsseite und der Blutseite eingestellt ist, wird in Übereinstimmung mit Fig. 7 zur Berechnung des Verteilungsvolumens mittels eines quantitativen Verfahrens, beispielsweise eines pH-Shift-Verfahrens bzw. pH-Verschiebungsverfahrens, die Konzentration eines gewünschten Analyten auf der Dialysierflüssigkeitsseite gemessen (Wert $C_1$). Da Stoffe aus dem Blut vollständig in die Dialysierflüssigkeit übertreten, ist bei gleichem Dialysierflüssigkeits- und Blutfluss deren Konzentration in der Dialysierflüssigkeit dieselbe wie diejenige im Blut des Patienten. Bei unterschiedlichen Flüssen gilt Gleichung 4. Gleichzeitig zum vorgenannten quantitativen Verfahren wird ein Extinktionssignal bzw. Extinktionswert gemessen oder wird ein bereits ermitteltes Extinktionssignal bzw. ein bereits ermittelter Extinktionswert aus beispielsweise dem pH-Shift-Verfahren übernommen. Sodann wird der Konzentrationswert über einen Umrechnungsfaktor oder eine Formelbeziehung mit dem Extinktionswert verknüpft bzw. korreliert. Als Nächstes wird das Extinktionssignal als Funktion der Therapiezeit gemessen und wird danach aus dem dann kontinuierlich vorliegenden Signal zu gewünschten Zeitpunkten ein Konzentrationssignal berechnet. Im Weiteren wird durch erneute Anwendung eines quantitativen Verfahrens die blutseitige Konzentration des Analyten unter der vorstehenden Bedingung einer Clearance von 100% als ein Wert $C_2$ gemessen. Sodann wird durch Bildung eines Integrals aus den berechneten Werten die entfernte Gesamtmasse m bestimmt. Mit den soweit ermittelten Größen und/oder Werten kann nun unter Verwendung der vorstehenden Gleichung (6) das Verteilungsvolumen V ermittelt werden.

Für den Fall, dass der berechnete Wert für $C_2$ mit dem gemessenen Wert nicht übereinstimmt, eignet sich die Messung von $C_2$ zur Erfassung und Feststellung beispielsweise etwaiger Transportbehinderungsmechanismen bzw. Transportprobleme, d.h. fluidbezogener Transportbeeinträchtigungen. Auch hierzu kann das vorstehend beschriebene Trackingverfahren herangezogen werden.

Fig. 8 zeigt eine Darstellung eines Signalverlaufs einer optischen Extinktionsmessung in Dialysierflüssigkeit sowie eine tatsächliche Entwicklung der blutseitigen Konzentration des Analyten im Falle eines Transportproblems an der Dialysatormembran, und veranschaulicht die wesentlichen Prozesse und Verfahrensschritte zur Erkennung von Transportproblemen in beispielsweise einem Dialysator während einer Therapie.

Unter der Voraussetzung, dass während einer Therapiebehandlung durch geeignetes Festlegen des Blutflusses (beispielsweise auf 50 ml/min) und des Dialysierflüssigkeitsflusses (beispielsweise auf 500 ml/min) eine Clearance von 100% zwischen der Dialysierflüssigkeitsseite und der Blutseite eingestellt ist, wird in Übereinstimmung mit Fig. 8 zur Berechnung des Verteilungsvolumens mittels eines quantitativen Verfahrens, beispielsweise eines pH-Shift-Verfahrens bzw. pH-Verschiebungsverfahrens, die Konzentration eines gewünschten Analyten auf der Dialysierflüssigkeitsseite gemessen (Wert $C_1$). Da Stoffe aus dem Blut vollständig in die Dialysierflüssigkeit übertreten, ist bei gleichem Dialysierflüssigkeits- und Blutfluss deren Konzentration in der Dialysierflüssigkeit dieselbe wie diejenige im Blut des Patienten. Bei unterschiedlichen Flüssen gilt wiederum Gleichung 4. Gleichzeitig zum vorgenannten quantitativen Verfahren wird ein Extinktionssignal bzw. Extinktionswert gemessen oder wird ein bereits ermitteltes Extinktionssignal bzw. ein bereits ermittelter Extinktionswert aus beispielsweise dem pH-Shift-Verfahren übernommen. Sodann wird der Konzentrationswert über einen Umrechnungsfaktor oder eine Formelbeziehung mit dem Extinktionswert verknüpft bzw. korreliert. Als Nächstes wird das Extinktionssignal als Funktion der Therapiezeit gemessen und wird danach aus dem dann kontinuierlich vorliegenden Signal zu gewünschten Zeitpunkten ein Konzentrationssignal berechnet. Im Weiteren wird durch erneute Anwendung eines quantitativen Verfahrens die blutseitige Konzentration des Analyten unter der vorstehenden Bedingung einer Clearance von 100% als ein Wert $C_2$ gemessen. Sodann wird der berechnete Wert für die Konzentration mit dem gemessenen Wert verglichen. Bei Gleichheit der miteinander verglichenen Werte wird auf das Nichtvorhandensein eines Transportproblems geschlossen. Bei Ungleichheit der miteinander verglichenen Werte wird auf ein bestehendes Transportproblem geschlossen.

Auf der Grundlage des Resultats des vorstehenden Verfahrens ist es möglich, die Reinigungsleistung während der Therapie sicherzustellen. Beispielsweise würde der üblicherweise dazu herangezogene Maßstab, der Kt/V-Wert, bei bestehenden Transportproblemen zur Feststellung einer besonders guten Reinigungsleistung führen, während tatsächlich die Reinigungsleistung verschlechtert ist, weil aufgrund der Transportbehinderungsmechanismen nicht ausreichend urämische Toxine entfernbar waren. Da durch das vorgenannte Verfahren die Reinigungsleistung jedoch korrekt beurteilt und damit sichergestellt werden kann, kann bei schlechter Reinigungsleistung das System eine Warnung ausgeben und daraufhin entweder der Dialysator gewechselt werden oder die Therapie mit verminderter Leistung so lange fortgesetzt werden, bis die entsprechende Dialysedosis erreicht ist.

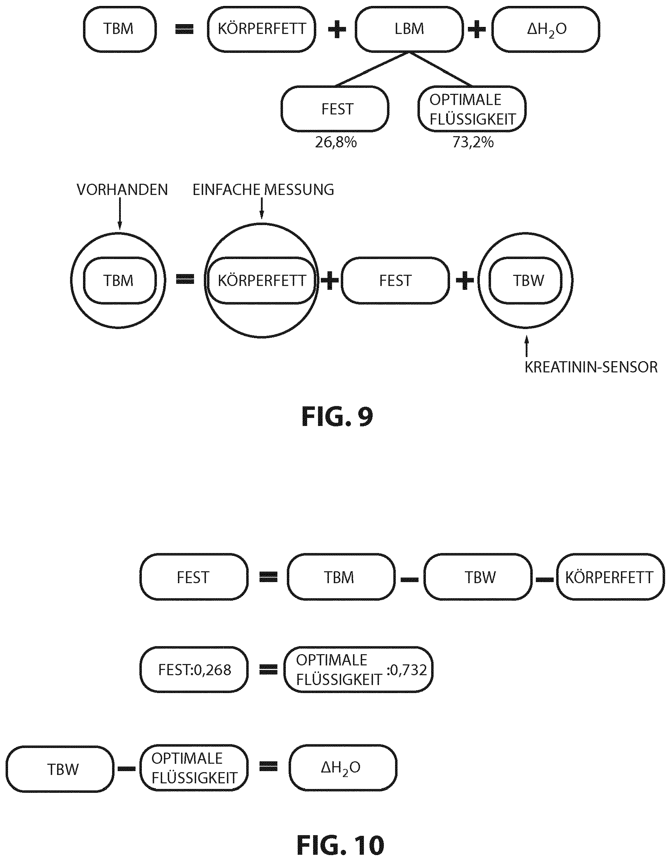

Fig. 9 zeigt eine schematische Darstellung in Bezug auf die Bestimmung von in einem Dialysepatienten vorhandenen überschüssigen Wassers sowie des Gesamtkörperwassers (Fluidmanagement).

In diesem Ausführungsbeispiel wird die in einem Dialysepatienten vorhandene überschüssige Menge an Wasser durch Bestimmen des Gesamtkörperwassers (TBW; Total Body Water), des Gesamtgewichts des Körpers (TBM; Total Body Mass), der Magermasse des Körpers, d.h. Körpergewicht minus Speicherfett (LBM; Lean Body Mass) bzw. des Fettgehalts oder Körperfetts (Bodyfat) des Dialysepatienten ermittelt. Grundlegend kann hierbei das Verteilungsvolumen V von beispielsweise Kreatinin dem Gesamtkörperwasser TBW gleichgesetzt werden. Das Verteilungsvolumen V kann mittels eines wie vorstehend beschriebenen, quantitativ messenden Sensors berechnet werden. Auf dieser Grundlage kann sodann das Gesamtgewicht des Körpers der Summe aus Körperfett, Magermasse LBM und überschüs- sigem Wasser $\Delta H_2O$ gleichgesetzt werden. Unter Anwendung eines an sich bekannten Zusammenhangs dahingehend, dass sich die Magermasse LBM für einen Menschen in ein festes Verhältnis von 26,8% Festbestandteilen und 73,2% optimaler Flüssigkeit auftrennen lässt, ist sodann die in Fig. 10 gezeigte Umformung der Darstellung in Fig. 9 in Bezug auf das Fluidmanagement herleitbar.

In anderen Worten ist durch eine Messung des Gesamtgewichts TBM, des Fettgehalts und des Gesamtkörperwassers TBW des Körpers die Menge des zu ultrafiltrierenden Wassers ermittelbar. Auf dieser Grundlage ist sodann die optimale Menge an Wasser berechenbar und kann in Kenntnis des Gesamtkörperwassers TBW die Menge an überschüssigem Wasser bestimmt werden.

Alternativ ist die Bestimmung der überschüssigen Menge an Wasser durch eine direkte Messung der Magermasse LBM möglich. Bei Verwendung eines Kreatinin-Sensors als quantitativen Sensor ist neben der Bestimmung des Gesamtkörperwassers TBW auch eine direkte Messung der Magermasse LBM durch Verwendung eines so genannten Kreatininkinetikmodells bzw. Creatinine Kinetic Model gemäß beispielsweise "Lean Body Mass Estimation by Creatinine Kinetics", Prakash R. Keshaviah et al. Journal of the American Society of Nephrology, Band 4, Nummer 7, 1994, möglich. Hierbei kann die Messung des Körperfettgehalts entfallen und wird die optimale Menge an Wasser direkt über die Magermasse LBM erhalten. Die überschüssige Menge an Wasser ist sodann durch Subtraktion der optimalen Menge an Wasser von dem Gesamtkörperwasser TBW ermittelbar.

Vorstehend wurde somit ein Verfahren zur Kalibrierung eines Messsignals und/oder zur Nachführung einer quantitativen Größe beschrieben. Das Verfahren beinhaltet das Vermessen eines mit einer Konzentration in einer Lösung vorliegenden Analyten, der eine vorbestimmte Abklingkinetik aufweist und das Erzeugen eines quasi-kontinuierlichen Messsignals mit einer zu der des Analyten zumindest entsprechenden Abklingkinetik. Die Abklingkinetik des Messsignals und die Abklingkinetik des Analyten werden unter Verwendung zumindest eines vorbestimmten Kalibrierpunkts beider Abklingkurven korreliert. Nachfolgende Konzentrationswerte des Analyten werden sodann aus dem Messsignal berechnet.

Es versteht sich, dass die Erfindung nicht auf die beschriebenen Ausführungsbeispiele und in deren Kontext angegebenen Zahlenwerte und Größenordnungen beschränkt ist, sondern dass sich innerhalb des durch die nachstehenden Ansprüche definierten Schutzumfangs für den Fachmann gleichwohl naheliegend Modifikationen und Äquivalente ergeben können.

**Patentansprüche**

1.  Verfahren zur Kalibrierung eines Messsignals und/oder zur Nachführung einer quantitativen Größe, beinhaltend:

    Vermessen eines mit einer Konzentration in einer Lösung vorliegenden Analyten, der eine vorbestimmte Abklingkinetik aufweist und Erzeugen eines kontinuierlichen Messsignals mit einer zu der des Analyten zumindest entsprechenden Abklingkinetik;
    Korrelieren der Abklingkinetik des Messsignals und der Abklingkinetik des Analyten unter Verwendung zumindest eines vorbestimmten Kalibrierpunkts beider Abklingkurven; und
    Berechnen nachfolgender Konzentrationswerte des Analyten aus dem Messsignal.

2.  Verfahren nach Anspruch 1, weiter beinhaltend:

    Auswählen eines für eine erste quantitative Größe erhaltenen ersten Messsignalverlaufs aus einer Vielzahl von für die erste quantitative Größe und für zumindest eine zweite quantitative Größe erhaltenen Messsignalverläufen;
    Durchführen einer Kalibrierung für zumindest einen Messwert eines zweiten Messsignalverlaufs bei der zweiten quantitativen Größe aus der Messung des entsprechenden Messwerts bei der ersten quantitativen Größe;
    Prüfen aller der bei der ersten quantitativen Größe und der zweiten quantitativen Größe erhaltenen Messwerte auf linearen oder zumindest hinreichend linearen Zusammenhang; und
    dann, wenn ein linearer oder zumindest hinreichend linearer Zusammenhang besteht, Berechnen zumindest eines nachfolgenden Messwerts für die zweite quantitative Größe aus dem ersten Messsignalverlauf für die erste quantitative Größe.

3.  Verfahren nach Anspruch 2, bei dem die erste quantitative Größe ein erster pH-Wert ist, die zweite quantitative Größe ein zweiter pH-Wert ist, und die Messwerte Extinktionswerte sind.

4.  Verfahren nach Anspruch 2 oder 3, bei dem dem ersten und dem zweiten Messsignalverlauf eine gleiche Wellenlänge von zur Ermittlung der Extinktionswerte verwendeten Analyselichts zugrunde liegt.

5.  Verfahren nach Anspruch 1, beinhaltend zur Berechnung eines Verteilungsvolumens:

    Messen der lösungsseitigen Konzentration eines in der Lösung vorhandenen gewünschten Analyten;
    Korrelieren eines Extinktionssignals und/oder zumindest eines Extinktionswerts mit der gemessenen Konzentration;
    Messen des Extinktionssignals und/oder zumindest eines Extinktionswerts als Funktion einer Zeit, die während einer Therapie verstreicht und Erhalten eines kontinuierlich vorliegenden Messsignals;
    Berechnen eines Konzentrationssignals und/oder zumindest eines Konzentrationswerts zu vorbestimmten, diskreten Zeitpunkten aus dem kontinuierlich vorliegenden Messsignal;
    Messen der blutseitigen Konzentration des Analyten;

Bestimmen einer entfernten Gesamtmasse; und

Ermitteln des Verteilungsvolumens aus der bestimmten Gesamtmasse, der lösungsseitigen Konzentration des Analyten und der blutseitigen Konzentration des Analyten.

6. Verfahren nach Anspruch 5, bei dem das Verteilungsvolumen in Übereinstimmung mit der Beziehung

$$V = m / (C1 - C2)$$

errechnet wird, worin C1 eine Konzentration des Analyten zu Beginn einer Therapiedauer und C2 eine Konzentration des Analyten am Ende der Therapiedauer ist und m die zwischen den beiden gemessenen Konzentrationen entfernte Gesamtmasse des Analyten und/oder dessen Integral ist.

7. Verfahren nach Anspruch 1, beinhaltend zur Erfassung einer fluidbezogenen Transportbeeinträchtigung:

Messen der lösungsseitigen Konzentration eines in der Lösung vorhandenen gewünschten Analyten;

Korrelieren eines Extinktionssignals und/oder zumindest eines Extinktionswerts mit der gemessenen Konzentration;

Messen des Extinktionssignals und/oder zumindest eines Extinktionswerts als Funktion einer Zeit, die während einer Therapie verstreicht und Erhalten eines kontinuierlich vorliegenden Messsignals;

Berechnen eines Konzentrationssignals und/oder zumindest eines Konzentrationswerts zu vorbestimmten, diskreten Zeitpunkten aus dem kontinuierlich vorliegenden Messsignal;

Vergleichen des berechneten Konzentrationssignals und/oder zumindest eines Konzentrationswerts mit der gemessenen Konzentration; und

Ermitteln eines Vorhandenseins einer Transportbeeinträchtigung bei Ungleichheit der miteinander verglichenen Signale und/oder Werte.

8. Verfahren nach einem der Ansprüche 5 bis 7, bei dem die Lösungsseite eine Dialysierflüssigkeitsseite ist.

9. Verfahren nach einem der Ansprüche 5 bis 8 mit einer Bedingung einer voreingestellten Clearance von 100% während der Therapiedauer.

10. Verfahren nach einem der Ansprüche 5 bis 9, bei dem sowohl die lösungsseitige Konzentration des Analyten als auch die blutseitige Konzentration des Analyten bei einer voreingestellten Clearance von 100% gemessen wird.

11. Verfahren nach einem der Ansprüche 5 bis 10, bei dem ein quantitatives Verfahren und/oder ein pH-Shift-Verfahren durchgeführt wird/werden.

12. Verfahren nach Anspruch 11, bei dem gleichzeitig zu dem quantitativen Verfahren ein Extinktionssignal und/oder zumindest ein Extinktionswert gemessen wird/werden.

13. Verfahren nach Anspruch 11, bei dem ein während der Durchführung des quantitativen Verfahrens bereits ermitteltes Extinktionssignal und/oder ein bereits ermittelter Extinktionswert übernommen wird/werden.

14. Verfahren nach einem der Ansprüche 5 bis 13, bei dem der Konzentrationswert über einen Umrechnungsfaktor und/oder eine Formelbeziehung mit dem Extinktionssignal und/oder dem zumindest einen Extinktionswert korreliert wird.

15. Verfahren nach einem der Ansprüche 5 bis 15, bei dem die entfernte Gesamtmasse durch Bildung eines Integrals aus den berechneten Werten bestimmt wird.

16. Verfahren nach Anspruch 5, weiter beinhaltend zur Bestimmung eines in einem Körper enthaltenen überschüssigen Wassers:

Bestimmen der Gesamtwassermenge, des Gesamtgewichts und des Fettgehalts des Körpers;

Ermitteln der Magermasse durch Subtrahieren des Fettgehalts und der Gesamtwassermenge von dem Gesamtgewicht;

Aufteilen der Magermasse in einen vorbestimmten Festbestandteil und einen vorbestimmten Flüssigkeitsanteil, wobei der Flüssigkeitsanteil als eine optimale Flüssigkeitsmenge angenommen wird;
Gleichsetzen des ermittelten Verteilungsvolumens und der Gesamtwassermenge des Körpers; und
Ermitteln der Menge überschüssigen Wassers durch Subtrahieren der optimalen Flüssigkeitsmenge von dem ermittelten Verteilungsvolumen.

17. Verfahren nach Anspruch 5, weiter beinhaltend zur Bestimmung eines in einem Körper enthaltenen überschüssigen Wassers:

Bestimmen der Gesamtwassermenge und des Gesamtgewichts des Körpers;
Ermitteln der Magermasse durch direkte Messung unter Verwendung eines vorbestimmten Kreatininkinetikmodells;
Aufteilen der Magermasse in einen vorbestimmten Festbestandteil und einen vorbestimmten Flüssigkeitsanteil, wobei der Flüssigkeitsanteil als eine optimale Flüssigkeitsmenge angenommen wird;
Gleichsetzen des ermittelten Verteilungsvolumens und der Gesamtwassermenge des Körpers; und
Ermitteln der Menge überschüssigen Wassers durch Subtrahieren der optimalen Flüssigkeitsmenge von dem ermittelten Verteilungsvolumen.

**FIG. 1**

**FIG. 2**

**FIG. 3**

**FIG. 4**

**FIG. 5**

- ● KALIBRIERPUNKT
- ○ BERECHNETER PUNKT
- — MESSSIGNAL
- ▨ INTEGRAL

SIGNAL

ZEIT

**FIG. 6**

**FIG. 7**

**FIG. 8**

TBM $=$ KÖRPERFETT $+$ LBM $+$ $\Delta H_2O$

FEST
26,8%

OPTIMALE
FLÜSSIGKEIT
73,2%

VORHANDEN     EINFACHE MESSUNG

TBM $=$ KÖRPERFETT $+$ FEST $+$ TBW

KREATININ-SENSOR

## FIG. 9

FEST $=$ TBM $-$ TBW $-$ KÖRPERFETT

FEST:0,268 $=$ OPTIMALE FLÜSSIGKEIT :0,732

TBW $-$ OPTIMALE FLÜSSIGKEIT $=$ $\Delta H_2O$

## FIG. 10

**Europäisches Patentamt**
**European Patent Office**
**Office européen des brevets**

# EUROPÄISCHER RECHERCHENBERICHT

Nummer der Anmeldung

EP 16 19 8632

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | JP 2013 027455 A (NIKKISO CO LTD) 7. Februar 2013 (2013-02-07) * Absatz [0011] - Absatz [0012]; Anspruch 1; Abbildungen 5-8 * ----- | 1-17 | INV. G01N21/00 G01N33/96 G01N33/70 |
| X | EP 2 290 371 A1 (HOFFMANN LA ROCHE [CH]; ROCHE DIAGNOSTICS GMBH [DE]) 2. März 2011 (2011-03-02) * Ansprüche 1,17,16,12,13 * ----- | 1-17 | |
| X | EP 2 942 614 A1 (BRAUN B AVITUM AG [DE]) 11. November 2015 (2015-11-11) * Absatz [0082] - Absatz [0092]; Ansprüche 12-14; Abbildung 6 * * Absatz [0028] * * Absatz [0040] - Absatz [0058] * ----- | 1-17 | |

RECHERCHIERTE SACHGEBIETE (IPC)

G01N

Der vorliegende Recherchenbericht wurde für alle Patentansprüche erstellt

| Recherchenort | Abschlußdatum der Recherche | Prüfer |
|---|---|---|
| München | 19. Dezember 2016 | Fleitmann, J |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

 

& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 16 19 8632

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

19-12-2016

| Im Recherchenbericht angeführtes Patentdokument | Datum der Veröffentlichung | Mitglied(er) der Patentfamilie | Datum der Veröffentlichung |
|---|---|---|---|
| JP 2013027455 A | 07-02-2013 | JP 5736268 B2<br>JP 2013027455 A | 17-06-2015<br>07-02-2013 |
| EP 2290371 A1 | 02-03-2011 | CN 102959401 A<br>EP 2290371 A1<br>EP 2470913 A1<br>HK 1177637 A1<br>US 2012191362 A1<br>US 2016070675 A1<br>WO 2011023760 A1 | 06-03-2013<br>02-03-2011<br>04-07-2012<br>09-10-2015<br>26-07-2012<br>10-03-2016<br>03-03-2011 |
| EP 2942614 A1 | 11-11-2015 | CN 105092504 A<br>CN 204882361 U<br>DE 102014106489 A1<br>EP 2942614 A1<br>US 2015323447 A1 | 25-11-2015<br>16-12-2015<br>12-11-2015<br>11-11-2015<br>12-11-2015 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **PRAKASH R. KESHAVIAH et al.** Lean Body Mass Estimation by Creatinine Kinetics. *Journal of the American Society of Nephrology,* 1994, vol. 4 (7